# EUROPÄISCHE PATENTSCHRIFT

(11) **EP 1 290 440 B1**
(45) Veröffentlichungstag und Bekanntmachung des Hinweises auf die Patenterteilung: **06.06.2012**
(21) Anmeldenummer: 01927552.8
(22) Anmeldetag: 14.05.2001
(51) Int. Cl.: G01N 33/36, G01N 27/22, G01N 21/89

(54) **VERFAHREN UND VORRICHTUNG ZUR ERKENNUNG VON FREMDSTOFFEN IN EINEM LÄNGSBEWEGTEN FADENFÖRMIGEN PRODUKT**
METHOD AND DEVICE FOR THE RECOGNITION OF IMPURITIES IN A LONGITUDINALLY MOVING THREAD-LIKE PRODUCT
PROCEDE ET DISPOSITIF DE DETECTION D'IMPURETES DANS UN PRODUIT DE TYPE FIL A DEPLACEMENT LONGITUDINAL

(30) Priorität: 31.05.2000 CH 10922000
(43) Veröffentlichungstag der Anmeldung: 12.03.2003
(73) Patentinhaber: Uster Technologies AG, 8610 Uster (CH)
(72) Erfinder: FURTER, Richard, CH-6300 Zug (CH)
(74) Vertreter: Pliska, Pavel
(86) Internationale Anmeldenummer: PCT/CH2001/000293
(87) Internationale Veröffentlichungsnummer: WO 2001/092875

(56) Entgegenhaltungen:
- EP-A- 0 545 129
- EP-A- 1 006 225
- EP-A1- 0 761 585
- WO-A-93/13407
- US-A- 5 054 317
- US-A- 5 383 017
- US-A- 5 832 709
- US-A- 5 915 279
- US-A- 5 926 267

## Beschreibung

Die Erfindung betrifft ein Verfahren und eine Vorrichtung zur Erkennung von Fremdstoffen in einem längsbewegten fadenförmigen Produkt aus textilen Fasern.

So ein Verfahren, sowie eine Vorrichtung, ist vom Dokument US 5,926,267 bekannt. Die beanspruchte Merkmale, die vom Dokument US 5,926,267 schon bekannt sind, befinden sich in der Präambel.

Aus der US 5,414,520 ist ein Verfahren und eine Vorrichtung zur Erkennung von Verunreinigungen, insbesondere Fremdfasem in langgestreckten textilen Gebilden bekannt. Dabei wird das Gebilde, beispielsweise ein Garn, in einem ersten Sensor durch Licht belichtet und das Ausmass des am Garn reflektierten Lichts gemessen. Damit werden insbesondere Verunreinigungen erkannt, deren Farbe, Struktur oder Oberflächenbeschaffenheit von derjenigen des Grundmaterials des Garns abweicht. Dabei erkennt man aber gleichzeitig auch Abweichungen der Masse oder des Durchmessers des Garns. Um diese Abweichungen auszuschalten, wird im selben oder in einem weiteren Sensor das Gebilde von der Gegenseite aus belichtet, so dass der Sensor nun die Abschattung durch das Gebilde misst. Kombiniert man nun das Signal, das durch die Reflexion und das Signal das durch die Abschattung entstanden ist, so entsteht ein Fremdstoffsignal, das vom Einfluss der Masse oder des Durchmessers des Gebildes befreit ist. Durch dieses Fremdstoffsignal wird üblicherweise das Messer eines Garnreinigers oder der Antrieb einer Spinnmaschine angesteuert, auf der Garnreiniger vorgesehen sind.

Ein Nachteil dieses bekannten Verfahrens und der Vorrichtung zur Erkennung von Verunreinigungen besteht darin, dass jedes Entfernen einer Verunreinigung einen Schnitt und auch ein Ansetzen der benachbarten Abschnitte eines Garns oder Bandes z.B. durch Spleissen zur Folge hat. Geschieht dies auf einer Spulmaschine, so muss dabei die Spulstelle stillgesetzt werden. Geschieht es auf einer Spinnmaschine, so wird die betreffende Spinnposition stillgesetzt. Das bedeutet, dass das Entfernen der Verunreinigungen im Produktionsprozess beispielsweise von textilen Garnen infolge solcher Stillstände, Einbussen bei der Leistung der betroffenen Maschinen verursacht. Insbesondere bei Spinnmaschinen bestehen diese Einbussen nicht nur aus Zeiten, die es braucht um das Garn zu trennen und wieder anzusetzen. Es können weitere Stillstandszeiten bewirkt werden, wenn gewartet werden muss, bis das Ansetzgerät, das üblicherweise viele Spinnstellen betreuen muss, verfügbar wird und die fragliche Spinnstelle erreicht hat. So ist es zwar einerseits wünschbar, Fremdstoffe oder Verunreinigungen zu entfernen, um Probleme bei der nachfolgenden Verarbeitung wie z.B. dem Weben, Färben oder Veredeln zu vermeiden. Es ist aber unerwünscht, dass dadurch die Leistung der Maschinen beeinträchtigt wird.

Aus diesen Gründen ist es wünschbar, dass sich beispielsweise der Hersteller eines textilen Zwischenproduktes wie z.B. Band, Garn usw. bewusst ist, ob und wieweit er Verunreinigungen oder Fremdstoffe im Band oder Garn entfernen will. Seine Möglichkeiten, eine Wahl zu treffen sind aber sehr beschränkt, weil Verfahren und Vorrichtungen entsprechend dem obengenannten Patent nur die Möglichkeit geben eine Schwelle zu setzen, jenseits welcher eine Verunreinigung eben entfernt wird oder nicht.

Es ist deshalb eine Aufgabe der vorliegenden Erfindung, wie sie in den Patentansprüchen gekennzeichnet ist, ein Verfahren und eine Vorrichtung zu schaffen, mit denen Verunreinigungen oder Fremdstoffe aufgrund wesentlich differenzierterer Kriterien erkannt und entfernt werden können.

Dies wird dadurch erreicht, dass am schnellbewegten Band oder Garn, wie beispielsweise bereits bekannt, ein erster Parameter mit einem Wellenfeld erfasst wird, wobei ein erstes Signal erzeugt wird, das allenfalls vorhandene Verunreinigungen oder Fremdstoffe anzeigt. Dieser erste Parameter erfasst vorzugsweise Reflexionsetgenschaften, wie sie an der Oberfläche des Produktes erfasst werden können. Zusätzlich soll nun noch ein weiterer Parameter in einem Feld am Band oder Garn erfasst werden, wobei ein zweites Signal erzeugt wird, das ebenfalls vorhandene Verunreinigungen oder Fremdstoffe anzeigt. Dieser zweite Parameter erfasst vorzugsweise Eigenschaften wie Masse oder Durchmesser des Garns oder Bandes, wie sie durch Messung der Abschattung eines Wellenfeldes oder der Änderung der Kapazität in einem elektrischen Feld ermittelt werden können. Als zweiter Parameter wird somit eine Grösse bestimmt, die wahlweise einer Gruppe von Grössen zugehört, wobei diese Gruppe die Masse und den Durchmesser eines Abschnittes des Produktes umfasst. Dem ersten und dem zweiten Signal, wobei beide Signale Verunreinigungen oder Fremdstoffe anzeigen, werden nun eigene Bewertungskriterien, beispielsweise Grenzwerte zugeordnet. Aus den Bewertungen des ersten und des zweiten Signals oder Parameters wird schliesslich eine bestimmte Art von Fremdstoffen ermittelt, wobei diese Art sich aus den gewählten Bewertungskriterien ergibt. Dabei ist es besonders vorteilhaft, die beiden Parameter in Feldern zu ermitteln, die sich durch ihre physikalischen Eigenschaften stark unterscheiden. So können sehr verschiedene Felder verwendet werden, wie z.B. Licht verschiedener Wellenlänge oder Licht und ein elektrisches Feld usw. Beide Parameter oder die daraus abgeleiteten Signale werden über eine vorgegebene Zeit beobachtet oder erfasst, eventuell integriert und erst anschliessend an diese Zeit mit den Bewertungsvorschriften verglichen oder daran gemessen. Diese Bewertungsvorschriften sind beispielsweise dazu angelegt, zwischen vegetabilen und nicht-vegetabilen Fremdstoffen oder Verunreinigungen im Produkt zu unterscheiden.

Die entsprechende Vorrichtung weist einen mit einem Wellenfeld arbeitenden ersten Sensor und einen zweiten Sensor, der mit einem Feld arbeitet, einen an den ersten und den zweiten Sensor angeschlossenen Prozessor, mit einem Speicher zur zeitlich begrenzten Speicherung der Signale aus dem ersten und dem zweiten Sensor und eine Software für den Prozessor auf, die Bewertungsvorschriften für die ersten und zweiten Signale vorgibt, mit denen aus dem ersten und dem zweiten Signal ein drittes Signal erzeugt wird, das mindestens zwei Arten der Fremdstoffe unterscheidet. Für den ersten Sensor ist als Wellenfeld vorzugsweise Licht einer bestimmten Farbe vorzusehen, für den zweiten Sensor ein elektrisches Feld.

Zwar ist aus der EP 0 401 600 bereits eine Vorrichtung zur Überwachung von Parametern eines laufenden fadenförmigen Garns bekannt, bei der ein kapazitiv und ein optisch arbeitender Sensor vorgesehen ist, die nebeneinander angeordnet sind und Messwerte abgeben, die vom Garn abgeleitet sind. Die Auswertung der beiden Signale erfolgt aber nicht im Hinblick auf die Erkennung von Verunreinigungen oder Fremdfasem, sondern im Hinblick auf die Verminderung der Abhängigkeit von Fremdeinflüssen wie Feuchtigkeit, Materialeinfluss, Abhängigkeit von der Form usw. bei der Messung der Gleichförmigkeit, oder zur Förderung der Selbstkontrolle. Eine differenzierte Erkennung von Verunreinigungen liegt aber nicht im Sinne dieser Veröffentlichung.

Auch aus der GB 2,095,828 ist ein Verfahren und eine Vorrichtung bekannt, die denjenigen aus der US 5,414,520 sehr ähnlich sind. Dies deshalb, weil auch hier die Reflexion und die Transmission von Licht an einem Faserverbund gemessen werden. Die Bildung des Verhältnisses der Signale aus der Reflexion und der Transmission führt zu einem Signal, welches faserige und vegetabile Fehler unterscheiden lässt. Durch weitere Untersuchungen dieser Signale hinsichtlich Angaben über die Grösse, die Durchlässigkeit für Licht und die Form kann noch eine feinere Klassierung der Fehler erfolgen. Diese sehr weitgehende Untersuchung von Fehlern ist aber für Vliese gedacht, die nicht schneller als etwa 1.5 m/min bewegt werden und die aus Wolle bestehen, wobei eben als Verunreinigungen solche Elemente gelten, die nicht aus der Wolle des Schafes stammen. Dagegen werden z. B. Garne beim Spinnen mit 200 - 400 m/min und beim Spulen mit bis zu 2500 m/min bewegt, so dass in solchen Fällen diese aufwendigen Untersuchungen nicht rechtzeitig durchzuführen sind.

Im Band oder Garn sind Fasern, die aus Kunststoff bestehen, Schnüre, menschliche und tierische Haare, Vogelfedern usw. die wir hier als nicht-vegetabile Verunreinigungen oder Fremdstoffe bezeichnen, besonders störend. Weniger störend sind beispielsweise bei Baumwolle als Grundmaterial für das Garn die Blattreste, Schalenteile, Samenteile usw. aus der Baumwolle, die wir hier als vegetabile Verunreinigungen oder Fremdstoffe bezeichnen. Mit anderen Worten bezeichnen wir als Vegetabilien solche Elemente, die aus der Baumwollpflanze stammen. Nicht als Vegetabilien bezeichnen wir Elemente oder Stoffe, die nicht aus der Baumwollpflanze stammen. Diese Elemente können aber trotzdem aus der Natur stammen, wie z.B. die Haare oder Vogelfedem.

Die durch die Erfindung erreichten Vorteile sind insbesondere darin zu sehen, dass durch eine gezielt differenzierte Erfassung und Ausscheidung der Fremdstoffe nach den obengenannten Gesichtspunkten einerseits die Nachteile in der nachfolgenden Verarbeitung und andererseits auch die Nachteile bei der Herstellung des aktuellen Zwischenproduktes, wie beispielsweise des Garns oder Bandes, vermieden werden können. Als wichtiges Beispiel kann eine Unterscheidung zwischen vegetabilen und nicht-vegetabilen Fremdstoffen gemacht werden, die bei der Erfassung der Verunreinigungen in der Form einer Bewertungsvorschrift für die erhaltenen Signale vorgegeben wird. Dies bedeutet beispielsweise, dass nur die nicht-vegetabilen Verunreinigungen entfernt und die vegetabilen Verunreinigungen im Garn belassen werden könnten. Eine solche Differenzierung ergibt den Vorteil, dass viele Verunreinigungen nicht aus dem Garn oder dem Band herausgeschnitten werden müssen und diese Verunreinigungen die nachfolgende Verarbeitung, z.B. das Färben nicht beeinträchtigen, da eben die vegetabilen Fremdstoffe die Farbe ebensogut wie die Baumwolle annehmen, oder da beim Bleichen allfällige ursprüngliche Farbunterschiede ausgeglichen werden. Eine solche Differenzierung ergibt aber auch den Vorteil, dass im Garn weniger Schnitte gemacht werden und somit die Leistung der Spinn- oder Spulmaschinen nicht so stark vermindert wird.

Im folgenden wird die Erfindung anhand eines Beispiels und mit Bezug auf die beiliegenden Figuren näher erläutert. Es zeigen:
Fig. 1 eine schematische Darstellung der erfindungsgemässen Vorrichtung,
Fig. 2 eine Darstellung von Signalen aus zwei Sensoren der Vorrichtung gemäss Fig. 1,
Fig. 3 eine Darstellung von Dimensionen von Verunreinigungen und von möglichen Grenzen für damit verbundene Signale aus den Sensoren,
Fig. 4 eine weitere Darstellung einer erfindungsgemässen Vorrichtung und
Fig. 5, 6, 7 und 8 je eine Darstellung von möglichen Bewertungskriterien wobei Fig.6 nicht Teil der Erfindung ist.

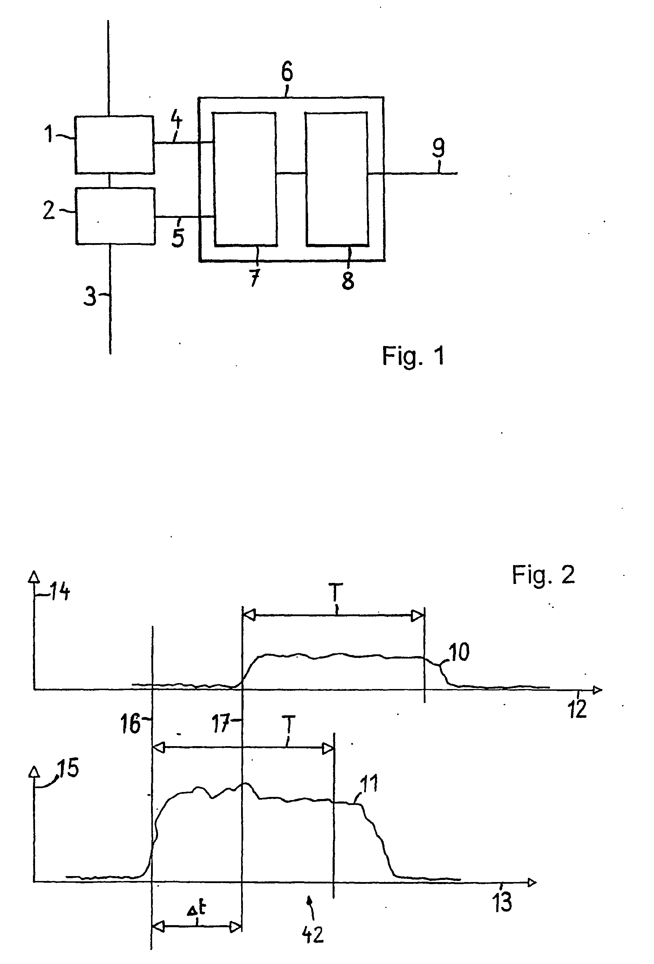

In Fig. 1 ist eine erfindungsgemässe Vorrichtung schematisch dargestellt. Sie besteht aus einem ersten Sensor 1, der beispielsweise als Fremdstoffsensor aufgebaut sein kann, wie er aus der EP 0 761 585 bekannt ist. Sie besteht ferner aus einem zweiten Sensor 2, der speziell auf die Masse oder den Durchmesser des Garns 3 anspricht. Ein solcher Sensor 2 ist beispielsweise aus der US 5,530,368 bekannt. Die Sensoren 1 und 2 sind Ober Verbindungen 4 und 5 mit einem Prozessor 6 verbunden. Dieser weist einen Speicher 7, einen Rechner 8 und einen Ausgang 9 für ein differenziertes Fremdstoffsignal auf. Der Prozessor 6 enthält eine Software, die Bewertungsvorschriften für die ersten und zweiten Signale vorgibt, mit denen aus dem ersten und dem zweiten Signal ein drittes Signal 9 erzeugt wird, das mindestens zwei Arten der Fremdstoffe unterscheidet.

Fig. 2 zeigt einen ersten Signalverlauf 10 aus dem ersten Sensor 1 und einen zweiten Signalverlauf 11, wie er aus dem zweiten Sensor 2 stammt. Beide Signalverläufe 10 und 11 sind über einer Zeitachse 12 und 13 aufgetragen. Ober der Achse 12, sind längs einer Achse 14 Werte für die Reflexion des Wellenfeldes am Garn 3 und längs einer Achse 15 sind Werte für die Masse oder den Durchmesser des Garns 3 aufgetragen. Markierungen 16 und 17 geben eine Zeitdifferenz Δt an, die dem Abstand der beiden Sensoren 1 und 2 zueinander und der Geschwindigkeit, mit der das Garn bewegt wird, proportional ist. Mit T ist eine Zeit bezeichnet, während der ein Signal gespeichert wird.

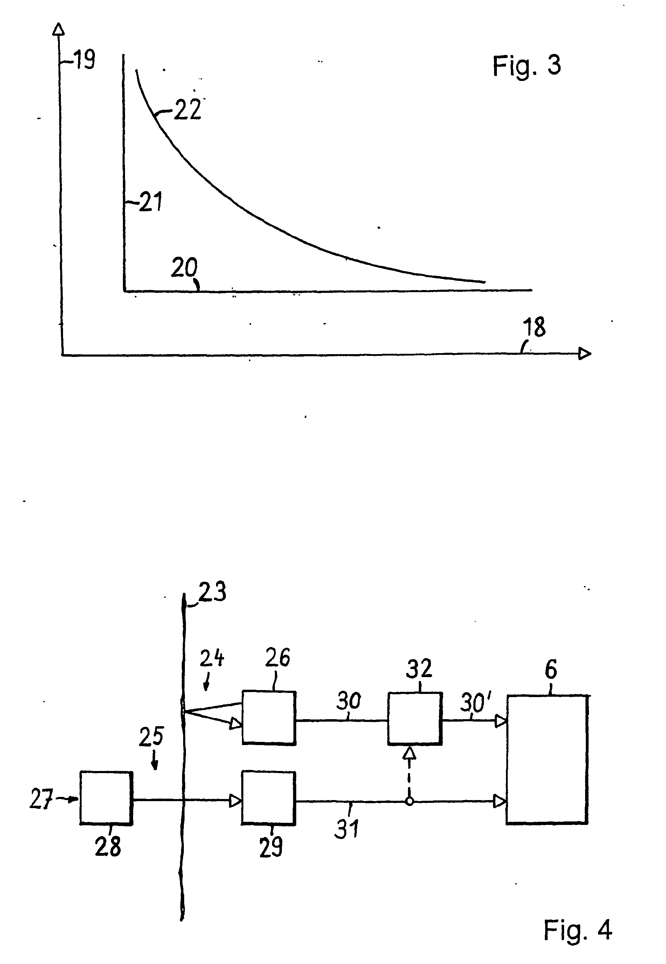

Fig. 3 zeigt eine an sich bekannte Möglichkeit, Garnfehler unabhängig davon ob sie mit Verunreinigungen in Zusammenhang stehen, gemäss ihrer Länge oder Dickenzunahme zu ordnen, indem man ihre Abmessung in das Feld einträgt, das sich zwischen Achsen 18 und 19 erstreckt. Längs der Achse 18 sind Werte für die Länge eines Fehlers und längs der Achse 19 sind Werte für die Ausdehnung des Fehlers quer zur Längsrichtung des Garns aufgetragen. Die Linien 20, 21 und 22 geben zwei aus vielen Möglichkeiten an, wie Grenzen für Fehler oder Verunreinigungen im Garn oder allgemein im Garn gelegt werden können. Typischerweise sind solche Verunreinigungen oder Fremdstoffe, die aufgrund ihrer Abmessungen oberhalb und rechts von den Linien 20, 21 oder 22 zu liegen kommen unannehmbar oder unerwünscht

Fig. 4 zeigt eine andere Ausführung der erfindungsgemässen Vorrichtung, hier mit einem Band oder Garn 23, das ein Wellenfeld 24 und ein weiteres Feld 25 durchquert. Man erkennt einen ersten Sensor 26 und einen zweiten Sensor 27, wobei der Sensor 26 z. B. einen Sender und einen Empfänger für Licht und der Sensor 27 Elemente 28, 29 aufweist, die beispielsweise entweder als Sender 28 und Empfänger 29 für Licht oder als Kondensatorelektroden 28, 29 ausgebildet sind. Über Leitungen 30 und 31 sind die beiden Sensoren 26, 27 mit einem Prozessor 6 verbunden. Ein wahlweise vorhandenes Element 32 kann der Kombination der Signale aus den Leitungen 30 und 31 dienen um in Leitung 30' ein korrigiertes Fremdstoffsignal zu erzeugen. Dies insbesondere dann, wenn der Sensor 27 für eine Durchlichtmessung ausgelegt ist.

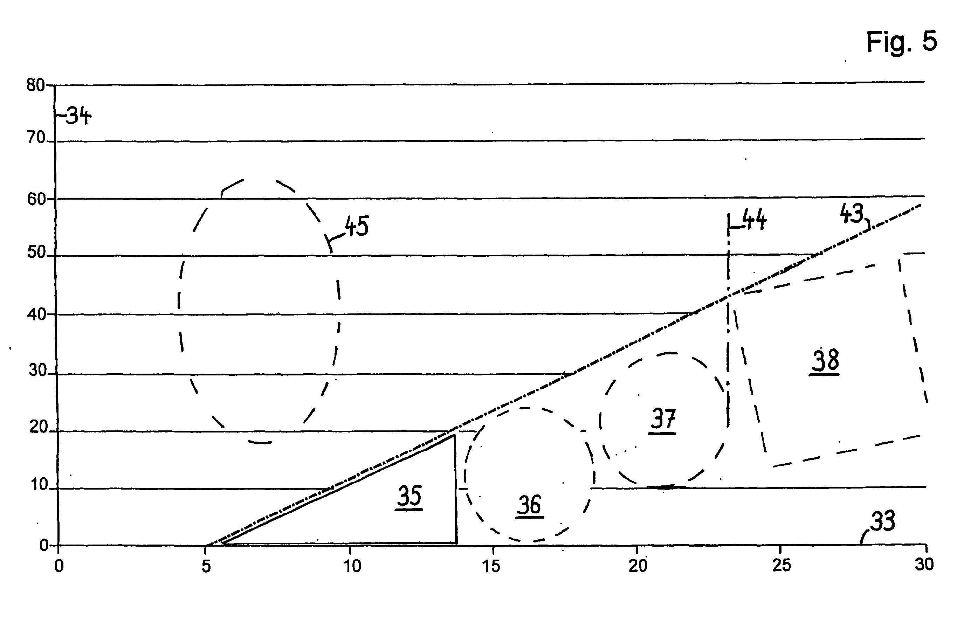

Fig. 5 zeigt eine Darstellung von Bewertungskriterien für eine differenzierte Beurteilung von Fremdstoffen oder Verunreinigungen. Dazu sind längs einer horizontalen Achse 33 Werte für die Signalabweichung in einem Wellenfeld wie z.B. dem Wellenfeld 24 und längs einer vertikalen Achse 34 Signalabweichungen in einem Feld wie z.B. dem Feld 25 aufgetragen. Beispielsweise betreffen die Zahlen auf der Achse 33 Werte für die Reflexion des Wellenfeldes am Produkt und die Zahlen auf der Achse 34 zeigen Werte für die Änderung der Kapazität in einem Kondensator oder der Transmission von Licht oder Wellen allgemein an. Hier stehen die Werte 0 für Mittelwerte oder Grundwerte und die nach rechts und nach oben angegebenen Zahlenwerte beziehen sich auf prozentuale Abweichungen oder insbesondere Zunahmen zu den Grundwerten. Mit 35 bis 38 sind Bereiche für die Signale aus den beiden Sensoren 1, 2 oder 26, 27 angegeben, in denen oft bestimmte Verunreinigungen oder Fremdstoffe liegen. Diese Bereiche 35 bis 38 sind durch Wertebereiche auf den beiden Achsen 33 und 34 gekennzeichnet. Der Bereich 35 betrifft beispielsweise Einzeifasern aus Kunststoff. Der Bereich 36 betrifft beispielsweise Streifen aus Kunststoff und Faserbündel. Der Bereich 37 betrifft beispielsweise menschliche und tierische Haare. Der Bereich 38 betrifft beispielsweise Tuchfragmente, fettige Faserbündel oder insgesamt grössere oder gröbere Verunreinigungen.

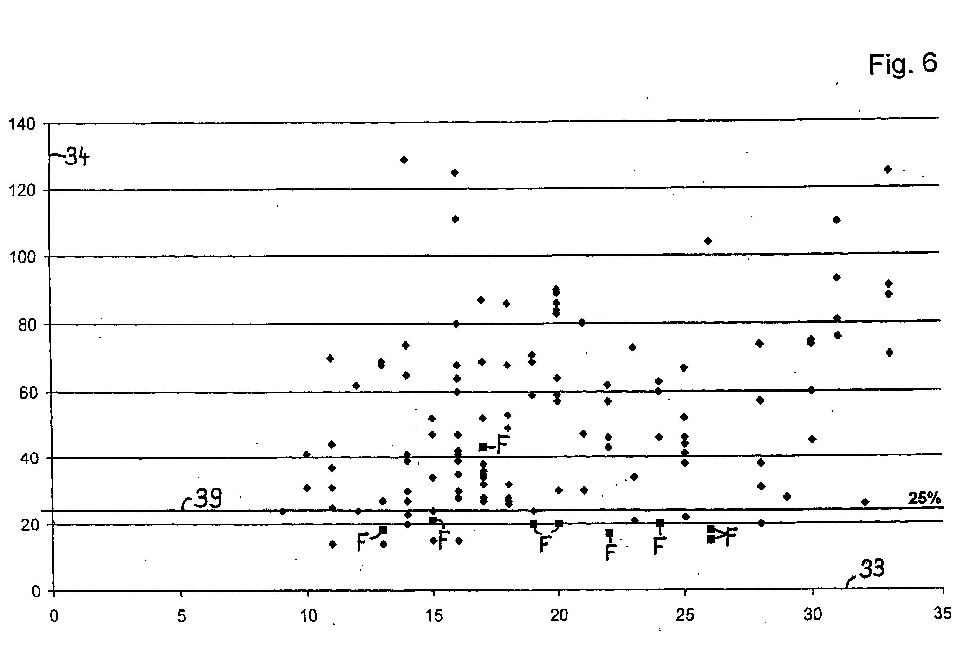

Fig. 6, die nicht Teil der Erfindung ist, zeigt eine Darstellung mit gemessenen Werten für Verunreinigungen, die über Achsen 33, 34 aufgetragen sind, wie diese bereits aus der Fig. 5 bekannt sind, hier aber eine andere Abstufung der Zahlenwerte aufweisen. Mit F sind unerwünschte fremde Fasern bezeichnet. Dazu ist ein Grenzwert 39 eingezeichnet, der bezogen auf die Signale, wie sie die Achse 34 darstellt, um 25% den Grundwert übersteigt.

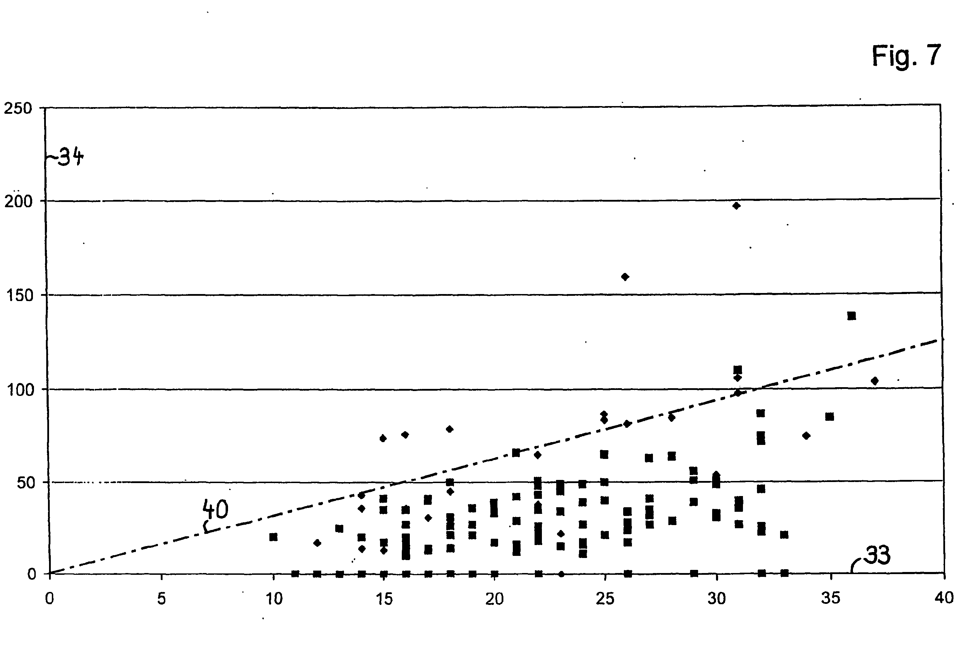

Fig. 7 zeigt eine weitere Darstellung mit gemessenen Werten für Verunreinigungen, die über Achsen 33, 34 aufgetragen sind, wie diese bereits aus der Fig. 5 bekannt sind, hier aber eine andere Abstufung der Zahlenwerte aufweisen. Unerwünschte fremde Fasern sind durch die rechteckigen Symbole bezeichnet. Dazu ist eine Grenze 40 eingezeichnet, die einer Funktion y = f(x) folgt, wenn man mit x die Werte längs der Achse 33 und mit y die Werte längs der Achse 34 bezeichnet.

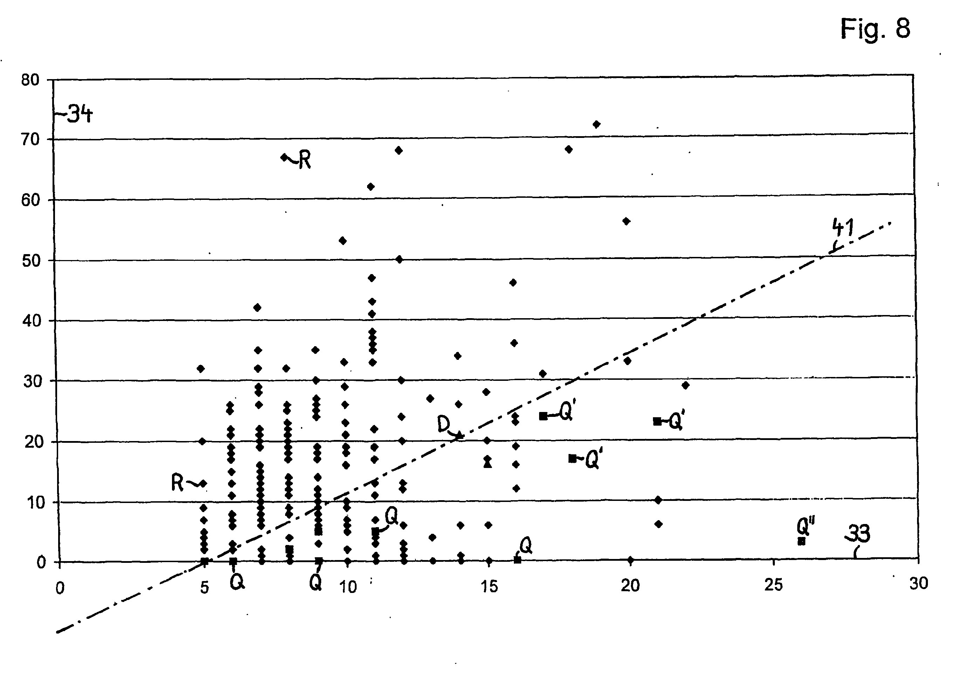

Fig. 8 zeigt eine weitere Darstellung mit gemessenen Werten für Verunreinigungen, die über Achsen 33, 34 aufgetragen sind, wie diese bereits aus der Fig. 5 bekannt sind, hier aber eine andere Abstufung der Zahlenwerte aufweisen. Verunreinigungen mit vegetabilem Ursprung sind hier mit kleinen Rhomben R, unerwünschte Fasern mit kleinen Quadraten Q, Reste von Kunststoffbändern mit kleinen Dreiecken D, schwarze Haare mit weiteren Quadraten Q' und Reste von Stoffen mit kleinen Quadraten Q" bezeichnet Dazu ist eine Grenze 41 eingezeichnet, die einer Funktion y = f(x) + X folgt, wenn man mit x die Werte längs der Achse 33 und mit y die Werte längs der Achse 34 bezeichnet.

Die Figuren 7 bis 8 zeigen somit Signale, wie sie in den Leitungen 4 und 5 auftreten können, wobei aber hier deren zeitlicher Verlauf nicht berücksichtigt ist. Der Umstand, dass die Werte der Signale vertikal übereinanderliegen ist nur dadurch bedingt, dass für die Werte der Achse 33 nur bestimmte diskrete Werte dargestellt sind.

Die Wirkungsweise der Vorrichtung und das Verfahren sind wie folgt:

Im ersten Sensor 1, 26 wird das Band oder das Garn 3, 23 zur Erfassung eines ersten Parameters, einem Wellenfeld 24, beispielsweise Licht ausgesetzt und es wird gemessen wieviel Licht oder Wellenenergie durch Reflexion am Produkt wieder erfasst werden kann. Dabei geht man davon aus, dass die Reflexion sich ändert, wenn Fremdstoffe im Sensor 1, 26 auftauchen und das Signal, das im Sensor 1, 26 entsteht, von einem Grundwert, der vom Grundmaterial bestimmt wird, abweicht. Beispielsweise ändert sich die Reflexion, wenn plötzlich andersfarbige Fasern oder Plastikteile im Garn auftreten. Das dabei entstehende Signal kann zusätzlich wie aus der US 5,414,520 bekannt, um einen Durchmesser- oder Masseeinfluss bereinigt werden und könnte einen Verlauf aufweisen, wie er in Fig. 2 mit 10 bezeichnet ist. Der erste Parameter ist hier somit die Intensität des reflektierten Wellenfeldes oder Lichtes, wie er beispielsweise von einem Grundwert ausgehend längs der Achse 33 in Fig. 5 bis 8 in Prozenten aufgezeichnet ist. Um den Einfluss der Masse des Bandes oder Garns im Signal in Leitung 30 (Fig. 4) zu neutralisieren, wird es im Element 32 in bekannter Weise mit dem Signal aus der Leitung 31 kombiniert.

Im zweiten Sensor 2, wird um eine Zeit Δt versetzt, beispielsweise in einem kapazitiv arbeitenden Sensor 2, 27 ein Signal erzeugt, das proportional zur Masse oder zum Durchmesser des Garns 3, 23 im erfassten Abschnitt ist. Das dabei entstehende Signal (Fig. 2) könnte einen Verlauf aufweisen, wie er in Fig. 2 mit 11 bezeichnet ist. In jedem Falle wird damit in Form einer Durchmesser- oder Masse-Zunahme ein weiterer Parameter am Garn erfasst, wie er beispielsweise von einem Grundwert ausgehend ebenfalls Längs der Achse 34 in Fig. 5 bis 8 in Prozenten aufgezeichnet ist.

Beide Signale werden nun über die Leitungen 4, 5, oder 30', 31 in den Speicher 7 des Prozessors 6 eingegeben, wo sie gespeichert werden. Die Zeit T, während der sie gespeichert werden, hängt von den verwendeten Bewertungskriterien ab. Beispielsweise davon, wann, ab welcher Länge oder Grenze ein Fremdstoff beginnt als störend empfunden zu werden. Es ist aus der Gamprüfung beispielsweise bekannt, dass sehr kurze Fehler auch dann nicht störend sind, wenn die Durchmesserzunahme durch den Fehler gross, z.B. 100% ist. So müssen für die ersten und zweiten Signale ebenfalls Grössen vorgegeben werden, oberhalb welchen ein störender Fremdstoff vorliegt und unterhalb welchen kein störender Fremdstoff vorliegt oder dieser einfach nicht wahrgenommen werden soll. Solche Grenzen sind in Fig. 3, 5 , 7 und 8 angegeben und zwar können sie für die Länge und die Dicken- oder Massenzunahme des Produktes durch den Fremdstoff und auch für das Ausmass und die Dauer einer von einem Grundwert abweichenden Reflexion vorgegeben werden. Nun soll auch diese Zeit T mindestens diejenige Zeit übersteigen, die der Geschwindigkeit des Garnes multipliziert mit der Länge entsprechend der Grenze (Linie 21) für die Länge des Signales bzw. der Verunreinigung entspricht. Diese Zeit T soll vorzugsweise zusätzlich noch um die Zeit Δt verlängert werden, so dass in einem Zeitabschnitt 42 zwei Signale für eine genügend lange Zeit gleichzeitig vorliegen.

Grundsätzlich sollen nur Signale den Bewertungskriterien unterworfen werden, die gewisse Grenzen 20, 21 oder 22 (Fig. 3) übersteigen, wobei die Grenze 22 einer Funktion folgt, die die beiden Grenzen für die Länge und die Dicke voneinander abhängig macht.

Ein einfaches Bewertungskriterium kann beispielsweise die nachfolgende Tabelle 1 darstellen.

**Tabelle 1:**

| | >Grenzwert | >Grenzwert |
|---|---|---|
| Signal 1 | ja | nein |
| Signal 2 | ja | nein |

Dabei kann beispielsweise bestimmt werden, dass Ereignisse, die beide Signale 1 und 2 die für jedes Signal individuell bestimmte Grenze überschreiten lassen, als die gesuchten Fremdstoffe gelten sollen. Dies kann mit Hilfe der Fig. 5 , 7 und 8 näher erklärt werden.

In der Darstellung gemäss Fig. 5 erkennt man Bereiche 35 bis 38 für Fremdstoffe oder Verunreinigungen, die möglicherweise alle unerwünscht sind. Trifft dies zu, so gilt als Bewertungskriterium eine Grenze, wie sie durch eine Linie 43 darstellbar ist In diesem Falle werden nur solche Verunreinigungen erkannt und eventuell ausgeschieden, die Signale erzeugen, die unterhalb der Linie 43 liegen; d.h. den Grundwert entsprechend Achse 34 überschreiten aber die Grenze gemäss Linie 43 nicht überschreiten. Eine mindestens 5%-ige Zunahme für Werte der Achse 33 ist ebenfalls gefordert. Möchte man aber in einer Variante, die nicht Teil der Erfindung ist, beispielsweise nur solche Verunreinigungen erkennen, die etwa im Bereich 38 liegen, so könnte eine Linie 44 als Grenze vorgesehen werden, statt der Linie 43. Vorzugsweise sind längs der Achse 33 Werte für die Reflexion im Wellenfeld und längs der Achse 34 Werte für die Zunahme der Kapazität in einem elektrischen Feld aufgezeichnet. Erfasst man die Reflexion am Band oder Garn beispielsweise mit Licht mit einer besonderen Farbe, so treten Verzerrungen auf, denn Verunreinigungen derselben Farbe werden nur ein zu geringes Signal abgeben, so dass sie sich beispielsweise scheinbar in einem Gebiet 45, statt jenseits der Linie 43 oder 44 befinden. Misst man den zweiten Parameter aber in einem elektrischen Feld, so erkennt man die Verunreinigung trotzdem gut an grossen Werten längs der Achse 34. Damit können Unzulänglichkeiten des einen Sensors durch den anderen Sensor ausgeglichen werden. Dazu würde dann eben auch gehören, dass man die Grenzen entsprechend verschiebt.

In Fig. 6 die nicht Teil der Erfindung ist, erkennt man, dass in diesem Falle mit dem Grenzwert 39,89% der unerwünschten Fremdstoffe erkannt werden, dass aber der Grenzwert 39 nur 12.8% der unschädlichen Verunreinigungen miterfasst. Dieses Resultat erhält man durch Auszählen der eingetragenen Ereignisse.

In Fig. 7 erkennt man, dass in diesem Falle mit der Grenze 40 fast alle unerwünschten Fremdstoffe erfasst werden können.

In Fig. 8 erkennt man, dass in diesem Falle mit der Grenze 41 alle unerwünschten Fremdstoffe erfasst werden können. Zudem werden etwa 16% an sich unschädliche Verunreinigungen miterfasst.

Für die Unterscheidung zwischen vegetabilen und nicht-vegetabilen Verunreinigungen, wird ein Parameter in einem elektrischen Feld erfasst. Bei der kapazitiven Erfassung ist es nämlich die Masse des Garns oder Fremdstoffes, die das Signal beeinflusst und dies tut sie viel stärker. Das erkennt man wenn man sich vorstellt wie gering sich eine Masseänderung auf den Durchmesser auswirkt. Beispielsweise ergibt bei einem zylindrischen Körper eine Zunahme des Durchmessers um 10% eine Zunahme der Masse um 21%. Man kann aber nicht nur diese Masseänderung betrachten um Fremdstoffe zu erkennen, denn diese beeinflussen auch die Oberfläche des Bandes oder Garns. Deshalb ist ein weiterer Parameter zu untersuchen und erst die gemeinsame Auswertung beider Parameter ergibt ein gutes Ergebnis. Wenn die gewünschte Unterscheidung betr. erwünschter oder geduldeter und unerwünschter Fremdstoffe vorliegt, kann mit dem Signal in Ausgang 9 (Fig. 1) die Trennstufe eines Reinigers für das Band oder Garn angesteuert werden.

## Patentansprüche

1. Verfahren zur Erkennung von Fremdstoffen in einem längsbewegten fadenförmigen Produkt (3) aus textilen Fasern, wobei
am Produkt ein erster Parameter in einem Wellenfeld erfasst wird und dabei ein erstes Signal (10) erzeugt wird, das allenfalls vorhandene Fremdstoffe anzeigt,
**dadurch gekennzeichnet, dass**
zusätzlich ein weiterer Parameter am Produkt in einem Feld erfasst wird und dabei ein zweites Signal (11) erzeugt wird, das allenfalls vorhandene Fremdstoffe anzeigt,
zur Erfassung des ersten Parameters die Abschattung und die Reflexion des Wellenfeldes am Produkt gemessen werden und dabei zwei Signale entstehen, die zusammen kombiniert das erste Signal (30') ergeben,
als zweiter Parameter eine Masse eines Abschnittes des Produktes bestimmt und die Masse kapazitiv erfasst wird,
dem ersten und dem zweiten Signal eigene Bewertungsvorschriften in Form von Grenzwerten zu deren Bewertung zugeordnet werden und
aus den bewerteten ersten und zweiten Signalen durch gemeinsame Auswertung eine Unterscheidung zwischen vegetabilen und anderen Fremdstoffen vorgenommen wird.

2. Verfahren nach Anspruch 1, **dadurch gekennzeichnet, dass** das erste und das zweite Signal über eine Zeit (T) gespeichert werden und dass die beiden gespeicherten Signale ausgehend von einer Bewertungsvorschrift bewertet werden und aus der Bewertung die Art des Fremdstoffes bestimmt wird.

3. Verfahren nach Anspruch 1, **dadurch gekennzeichnet, dass** als Wellenfeld sichtbares Licht verwendet wird.

4. Vorrichtung zur Durchführung des Verfahrens nach Anspruch 1, beinhaltend einen mit einem Wellenfeld arbeitenden ersten Sensor (1) , **dadurch gekennzeichnet, dass** die Vorrichtung Weiter einen mit einem Feld arbeitenden zweiten Sensor (2) beinhaltet,
im ersten Sensor (1) das Produkt (3) Licht ausgesetzt wird, gemessen wird, wie viel Licht durch Reflexion am Produkt (3) wieder erfasst wird und das dabei entstehende Signal zusätzlich um einen Durchmessereinfluss bereinigt wird, der zweite Sensor (2) kapazitiv arbeitet und ein Signal erzeugt, das proportional zur Masse des Produktes (3) im erfassten Abschnitt ist,
die Vorrichtung einen an den ersten und den zweiten Sensor angeschlossenen Prozessor (6) mit einem Speicher (7) zur zeitlich begrenzten Speicherung der Signale aus dem ersten und dem zweiten Sensor beinhaltet und
die Vorrichtung eine Software für den Prozessor, die Bewertungsvorschriften in Form von Grenzwerten für die ersten und zweiten Signale vorgibt, mit denen aus dem ersten und dem zweiten Signal durch gemeinsame Auswertung ein drittes Signal erzeugt wird, das mindestens zwei Arten der Fremdstoffe, vegetabile und andere, unterscheidet, beinhaltet.

## Claims

1. A method for detecting impurities in a longitudinally moved thread-like product (3) made of textile fibers, wherein
a first parameter on the product is detected in a wave field and a first signal (10) is generated thereby which is indicative of potentially present impurities,
**characterized in that**
additionally a further parameter is detected on the product in a field and a second signal (11) is generated thereby which is indicative of potentially present impurities,
the shading and the reflection of the wave field are measured on the product for detecting the first parameter and two signals are generated thereby which jointly combined result in the first signal (30'),
a mass of a section of the product is determined as a second parameter and the mass is detected in a capacitive manner,
the first and the second signal are assigned separate evaluation specifications in form of threshold values for their evaluation, and
a differentiation between vegetable and other impurities is performed from the evaluated first and second signals by common evaluation.

2. The method according to claim 1, **characterized in that** the first and the second signal are stored over a time (T) and both stored signals are evaluated on the basis of an evaluation specification and the type of the impurity is determined from the evaluation.

3. The method according to claim 1, **characterized in that** visible light is used as the wave field.

4. An apparatus for performing the method according to claim 1, containing a first sensor (1) operating with a wave field,
**characterized in that**
the apparatus further contains a second sensor (2) operating with a field,
the product (3) is subjected to light in the first sensor (1), the amount of light by reflection on the product is detected again, and the signal generated thereby is additionally adjusted by a diametral influence,
the second sensor (2) operates in a capacitive manner and generates a signal which is proportional to the mass of the product (3) in the detected section,
the apparatus contains a processor (6) connected to the first and second sensor with a memory (7) for a temporally limited storage of the signals of the first and second sensor, and
the apparatus contains a software for the processor which predetermines evaluation specifications in form of threshold values for the first and second signals, with which a third signal is generated from the first and second signal by common evaluation, which third signal distinguishes between at least two types of impurities, vegetable and others.

## Revendications

1. Procédé pour la détection de corps étrangers dans un produit filamenteux (3) fait de fibres textiles déplacé longitudinalement, dans lequel un premier paramètre du produit est acquis dans un champ d'ondes et un premier signal (10) indiquant des corps étrangers éventuellement présents est ainsi produit,
**caractérisé en ce que**
un deuxième paramètre du produit est en outre acquis dans un champ et un deuxième signal (11) indiquant des corps étrangers éventuellement présents est ainsi produit,
pour la détection du premier paramètre, l'ombre portée et la réflexion du champ d'ondes sur le produit sont mesurées et il se forme ainsi deux signaux qui donnent, une fois combinés, le premier signal (30'),
pour le deuxième paramètre, une masse d'un segment du produit est déterminée et la masse est acquise de façon capacitive,
des consignes d'analyse formées par des limites d'analyse sont attribuées au premier signal et au deuxième, et
une différenciation entre des corps étrangers végétaux et autres est effectuée à partir du premier signal et du deuxième par une analyse conjointe.

2. Procédé selon la revendication 1, **caractérisé en ce que** le premier signal et le deuxième sont enregistrés pendant un temps (T) et **en ce que** les deux signaux enregistrés sont analysés à partir d'une consigne d'analyse et la nature du corps étranger est déterminée à partir de l'analyse.

3. Procédé selon la revendication 1, **caractérisé en ce que** le champ d'ondes utilisé est de la lumière visible.

4. Dispositif pour la mise en oeuvre du procédé selon la revendication 1, comprenant un capteur (1) fonctionnant avec un champ d'ondes,
**caractérisé en ce que**
le dispositif comprend en outre un deuxième capteur (2) fonctionnant avec un champ,
le produit (3) est exposé à de la lumière dans le premier capteur (1), la quantité de lumière acquise par réflexion sur le produit (3) est mesurée et le signal ainsi produit est en outre corrigé de l'influence du diamètre,
le deuxième capteur (2) fonctionne de façon capacitive et produit un signal proportionnel à la masse du produit (3) dans le segment d'acquisition,
le dispositif comprend un processeur (6) connecté au premier capteur et au deuxième avec une mémoire (7) pour l'enregistrement limité dans le temps des signaux du premier capteur et du deuxième, et
le dispositif comporte un logiciel pour le processeur qui définit des consignes d'analyse sous forme de limites pour le premier signal et le deuxième, avec lesquelles un troisième signal contenant au moins deux types de corps étrangers, végétaux et autres, est produit par analyse conjointe à partir du premier signal et du deuxième.
